# EUROPEAN PATENT SPECIFICATION

(11) **EP 1 003 506 B1**
(45) Date of publication and mention of the grant of the patent: **26.10.2005**
(21) Application number: 98923815.9
(22) Date of filing: 27.05.1998
(51) Int. Cl.: A61K 31/4164, A61K 31/4178, A61L 9/12

(54) **USE OF EPROSARTAN IN THE TREATMENT OF ISOLATED SYSTOLIC HYPERTENSION**
VERWENDUNG VON EPROSARTAN ZUR BEHANDLUNG VON ISOLIERTEM SYSTOLISCHEM BLUTHOCHDRUCKS
UTILISATION DE L'EPROSARTAN DANS LE TRAITEMENT DE L'HYPERTENSION SYSTOLIQUE ISOLEE

(30) Priority: 27.05.1997 US 47800 P
(43) Date of publication of application: 31.05.2000
(73) Proprietor: SMITHKLINE BEECHAM CORPORATION, Philadelphia, Pennsylvania 19101 (US)
(72) Inventor: BROOKS, David, P., West Chester, PA 19380 (US); FEUERSTEIN, Giora, Z., Wynnewood, PA 19096 (US); OHLSTEIN, Eliot, H., Glenmoore, PA 19343 (US); RUFFOLO, Robert, R., Jr., Spring City, PA 19475 (US)
(74) Representative: Blakey, Alison Jane
(86) International application number: PCT/US1998/010794
(87) International publication number: WO 1998/053816

(56) References cited:
- US-A- 5 185 351
- US-A- 5 418 250
- W.B. WHITE ET AL.: "Evaluation of the Angiotensin II Receptor Antagonist, Eprosratan, by Casual and Ambulatory BP Monitoring" CLINICAL RESEARCH, vol. 42, no. 3, 1994, page 447A XP002148354
- SCHOLZE J.: "Angiotensin II Receptor Antagonists: Clinical Relevance." INTERNIST, vol. 37, no. 6, 1996, pages 636-642, XP000918723
- WEXLER R R ET AL: "NONPEPTIDE ANGIOTENSIN II RECEPTOR ANTAGONISTS: THE NEXT GENERATIONIN ANTIHYPERTENSIVE THERAPY" JOURNAL OF MEDICINAL CHEMISTRY,US,AMERICAN CHEMICAL SOCIETY. WASHINGTON, vol. 39, no. 3, 2 February 1996 (1996-02-02), pages 625-656, XP002922745 ISSN: 0022-2623
- KOSTIS J.B.: "Angiotensin II-Receptor Blockers: Profile of a New Drug Class for Antihypertensive Therapy." AMERICAN JOURNAL OF GERIATRIC CARDIOLOGY, vol. 5, no. 6, 1996, pages 11-19, XP000918755
- HARRISON T.R.: 'Harrison s: Principles of Internal Medicine', 1994, MCGRAW-HILL, INC., USA

## Description

### Field of the Invention

This invention relates to the use of eprosartan, which is (E)-α-[2-n-butyl-1-[(4-carboxyphenyl)methyl]-1H-imidazol-5-yl]methylene-2-thiophenepropionic acid monomethanesulfonate, to treat isolated systolic hypertension.

### Background of the Invention

The renin-angiotensin system plays a major role in the long-term control of blood pressure. Inhibition of this system with ACE inhibitors, and more recently angiotensin II (AII) receptor antagonists, has provided important therapeutics for the treatment of hypertension. Additionally, it is known that the sympathetic nervous system plays an important role in blood pressure control. Indeed, sympathetic nervous system activity is a major determinant of systolic hypertension, which is now recognized as a significant risk factor for cardiovascular disease.

The compound (E)-α-[2-n-butyl-1-[(4-carboxyphenyl)methyl]-1H-imidazol-5-yl]methylene-2-thiophenepropionic acid monomethanesulfonate is known by the name "eprosartan" and is the subject of U.S. Patent No. 5,185,351 (the'351 patent), issued February 9, 1993. This compound is a nonpeptide AII receptor antagonist.

US5444080 discloses substituted 1-H-imidazol-5-ylialkanoic acids having angiotensin II receptor agonist activity, useful in the treatment of hypotension and associated diseases.

An abstract in Clinical Research (1994) 42(3) p 447A describes the evaluation of eprosartan by casual and ambulatory BP monitoring.

Internist (1996) 37(6) pp. 636-642 discusses the clinical relevance of angiotensin II receptor antagonists including irbesatan, valsartan, eprosartan and losartan.

J Medicinal Chemistry (1996) 39(3) pp. 625-656 discusses non-peptide angiotensin II receptor antagonists, such as eprosartan and losartan.

American J. Geriatric Cardiology (1996) 5(6) pp 11-19 discusses the profile of angiotensin II-receptor blockers such as losartan for antihypertensive therapy.

Harrison's Principles Internal Medicine, 13^{th} edition (1994), pp 1116-1117 discusses hypertensive vascular disease and includes a classification of arterial hypertension, defined as systolic hypertension with wide pulse pressure and systolic and diastolic hypertension (increased peripheral and vascular resistance).

Surprisingly, it has been found that eprosartan produced significant inhibition of the AII-induced enhancement of the pressor response to sympathetic nervous system activity. This result is surprising since other nonpeptide AII receptor antagonists, for example losartan, valsartan, and irbesartan, did not produce significant inhibition of this pressor response. Thus, eprosaran may be useful in the treatment of isolated systolic hypertension.

### Summary of the Invention

The present invention provides the use of eprosartan in the manufacture of a medicament for the treatment of isolated systolic hypertension.

### Detailed Description of the Invention

Eprosartan, which is (E)-α-[2-n-butyl-1-[(4-carboxyphenyl)methyl]-1H-imidazol-5-yl]methylene-2-thiophenepropionic acid monomethanesulfonate, has the following structure:

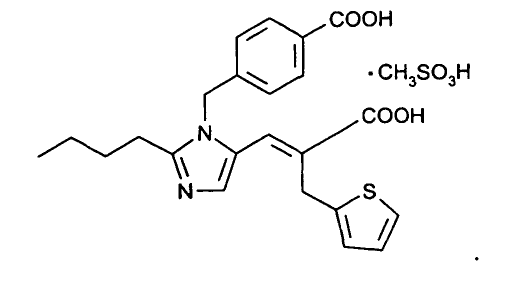

Eprosartan is claimed in U.S. Patent No. 5,185,351 (the'351 patent). Reference should be made to said patent for its full disclosure, including the methods of preparing this compound.

In accordance with the present invention, it has been unexpectedly found that eprosartan produced significant inhibition of the AII-induced enhancement of the pressor response to sympathetic nervous system activity. This result is surprising, since other nonpeptide AII receptor antagonists, for example losartan, valsartan, and irbesartan, did not produce significant inhibition of this pressor response. Thus, eprosaran may be useful in the treatment of isolated systolic hypertension.

Systolic hypertension is a major risk factor for cardiovascular disease and is present in a majority of hypertensive patients. It is well known that the sympathetic nervous system plays pivotal role in determining systolic blood pressure. Since the renin-angiotensin system can enhance sympathetic nervous system activity, it is possible that the antihypertensive activity of the newly developed AII receptor antagonists may involve prejunctional AII receptors, in addition to blockade of vascular AII receptors.

According to the instant invention, a number of nonpeptide AII receptors antagonists were evaluated for their ability to block prejunctional AII receptors and sympathetic outflow. Blockade of AII receptors with eprosartan resulted in a significant inhibition of the pressor response to spinal cord stimulation in the pithed rat. Several other nonpeptide AII receptor antagonists failed to produce this response. Thus, administration of losartan, valsartan, and irbesartan at equivalent and effective doses did not have any effect on the frequency-response curves. These data suggest that there may be differential effects between eprosartan and losartan, valsartan and irbesartan on prejunctional AII receptors. Thus, eprosartan, but not other nonpeptide AII receptor antagonists, may be effective at treating isolated systolic hypertension.

In the therapeutic use for the treatment of isolated systolic hypertension, eprosartan is incorporated into standard pharmaceutical compositions. It can be administered orally, parenterally, rectally, topically or transdermally.

Eprosartan can be formulated as a liquid, for example a syrup, suspension or emulsion, in a tablet, capsule or lozenge.

A liquid formulation will generally consist of a suspension or solution of eprosartan in a suitable liquid carrier(s) for example, ethanol, glycerine, non-aqueous solvent, for example, polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueious gums, celluloses, silicates or oils and the dispersion or suspension then filled into a soft gelatin capsule.

Eprosartan when administered parenterally (i.e. by injection of infusion) can be formulated as a solution or a suspension.

A composition for parenteral administration will generally consist of a solution or suspension of eprosartan in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository composition comprises eprosartan with a binding and/or lubricating agent such as polymeric glycols, gelatins or coca butter or other low melting vegetable or synthetic waxes or fats.

A typical transdermal formulation comprises a conventional aqueous or non-aqueous vehicle, for example, a cream, ointment lotion or paste or in the form of a medicated plaster, patch or membrane.

For topical administration, the pharmaceutical composition adapted includes solutions, suspensions, ointments, and solid inserts. Typical pharmaceutically acceptable carriers are, for example, water, mixtures of water and water-miscible solvents such as lower alkanols or vegetable oils, and water soluble ophthalmologically acceptable non-toxic polymers, for example, cellulose derivatives such as methyl cellulose. The pharmaceutical preparation may also contain non-toxic auxiliary substances such as emulsifying, preserving, wetting, and bodying agents, as for example, polyethylene glycols; antibacterial components such as quaternary ammonium compounds; buffering ingredients such as alkali metal chloride; antioxidants such as sodium metabisulfite; and other conventional ingredients such as sorbitan monolaurate.

The present invention provides a pharmaceutical composition which comprises eprosartan and a pharmaceutically acceptable carrier. The pharmaceutical composition may be adapted for oral administration. This composition is presented as a unit dose pharmaceutical composition containing from 50 mg to 1.0 g of eprosartyan, preferably from 200 to 400 mg. Such a composition is normally taken from 1 to 4 times daily, preferably from 1 to 2 times daily. The preferred unit dosage forms include tablets or capsules. The compositions of this invention may be formulated by conventional methods of admixture such as blending, filling and compressing. Suitable pharmaceutically acceptable carriers for use in this invention include diluents, fillers, binders and disintegrants.

No unacceptable toxicological effects are expected when eprosartan is administered in accordance with the present invention.

The following example is illustrative of the instant invention.

### Example 1

### Materials and Methods

### Spinal cord-stimulated pithed rats

Normotensive male Sprague-Dawley rats (300-350 gm) were anesthetized with Brevital (10 mg/kg, i.v.), a tracheostomy was performed and the rats were then pithed by inserting a steel rod (1.5 mm in diameter) through the orbit and foramen magnum into the spinal cord. Immediately after pithing, rats were ventilated artificially with room air using a rodent respirator at a frequency of 60 cycles/min with a volume of 2 ml/100 gm body weight. The pithing rod was insulated except for a 6-cm section distal from the tip. Body temperature was maintained at 37-38°C by a thermostatic heating pad. Animals were treated with tubocurararine (1 mg/kg, i.v) and atropine (1 mg/kg, i.v.), to prevent muscle movement during spinal cord stimulation and parasympathetic effects, respectively. Systemic arterial blood pressure was measured from the right carotid artery by a Statham P23 pressure transducer and recorded on a Grass polygraph. The left jugular vein was cannulated for i.v. administration of drugs.

Stimulation of sympathetic vasomotor outflow was accomplished by a consecutive train of stimulation (50 V, 1 msec, 0.3-5.0 Hz) which was delivered for 15 sec at each frequency. Drugs were administered at 0.3 mg/kg, i.v. 10 min before the initiation of a second frequency-response curve. For all the compounds evaluated, this dose provided effective blockade of the pressor response to exogenous AII (100 ng/kg, i.v.). Each rat served as its own control.

### Data analyses and statistics

All data are shown as the means ± S.E.M. of the number (n) of observations. Statistical significance of the differences between drug-treated animals and vehicle-control animals was tested by a one-way analysis of variance with a P valued of 0.05 accepted as significant.

### Drugs

All solutions were prepared daily. The following drugs were used: AII, Sar¹,Ile⁸[AII], atropine sulfate, (+)-tubocurarine chloride (Sigma Chemical Co.), eprosartan (U.S. Patent No. 5,185,351, issued February 9, 1993), losartan (U.S. Patent No. 5,138,069, issued August 11, 1992), valsartan (U.S. Patent No. 5,399,578, issued March 21, 1995) and irbesartan (U.S. Patent No. 5,270,317, issued December 14, 1993).

### Results

Stimulation of the thoracolumbar sympathetic outflow in pithed rats produced frequency-dependent pressor responses. Administration of saline vehicle did not produce significant effects on the frequency-response curve. Continuous infusion of a sub-pressor dose of AII (40 ng/kg/min) produced significant leftward shifts of the frequency-response curve, indicative of potentiation of sympathetic nervous system function. In contrast, continuous infusion of the peptide AII receptor antagonist Sar¹-Ile⁸[AII] (10 ug/kg/min) significantly inhibited the increase in pressor response to spinal cord stimulation.

The effects of several nonpeptide AII receptor antagonist were evaluated. Eprosartan (0.3 mg/kg, i.v.) produced significant inhibition of the pressor responses mediated by sympathetic nervous system activation. In contrast, neither losartan, valsartan, or irbesartan produced significant inhibition of the pressor responses mediated by spinal cord stimulation.

## Claims

1. The use of eprosartan in the manufacture of a medicament for the treatment of isolated systolic hypertension.

2. The use according to claim 1 wherein the medicament is in the form of a tablet or capsule.

## Patentansprüche

1. Verwendung von Eprosartan bei der Herstellung eines Medikaments zur Behandlung von isoliertem systolischen Bluthochdruck.

2. Verwendung gemäß Anspruch 1, wobei das Medikament in Tabletten- oder Kapselform vorliegt.

## Revendications

1. Utilisation d'éprosartan dans la production d'un médicament destiné au traitement de l'hypertension systolique isolée.

2. Utilisation suivant la revendication 1, dans laquelle le médicament est sous forme d'un comprimé ou d'une capsule.
